(19) Europäisches Patentamt
European Patent Office
Office européen des brevets

(11) Veröffentlichungsnummer: **0 056 087**
**B1**

# (12) EUROPÄISCHE PATENTSCHRIFT

(45) Veröffentlichungstag der Patentschrift: **06.06.84**

(51) Int. Cl.³: **C 07 D 233/60**, C 07 D 249/08, A 01 N 43/50

(21) Anmeldenummer: **81108806.1**

(22) Anmeldetag: **23.10.81**

(54) **Verwendung von Wachstumsregulierenden alpha-Azolylglykolen.**

(30) Priorität: **18.12.80 DE 3047726**

(43) Veröffentlichungstag der Anmeldung: **21.07.82 Patentblatt 82/29**

(45) Bekanntmachung des Hinweises auf die Patenterteilung: **06.06.84 Patentblatt 84/23**

(84) Benannte Vertragsstaaten: **AT BE CH DE FR GB IT LI LU NL SE**

(56) Entgegenhaltungen:
**EP - A - 0 021 345**
**DE - A - 2 324 424**
**DE - A - 2 325 156**

(73) Patentinhaber: **BASF Aktiengesellschaft, Carl-Bosch-Strasse 38, D-6700 Ludwigshafen (DE)**

(72) Erfinder: **Zeeh, Bernd, Dr., Thorwaldsenstrasse 5, D-6700 Ludwigshafen (DE)**
Erfinder: **Goetz, Norbert, Dr., Schoefferstrasse 25, D-6520 Worms (DE)**
Erfinder: **Ammermann, Eberhard, Dr., Sachsenstrasse 3, D-6700 Ludwigshafen (DE)**
Erfinder: **Jung, Johann, Dr. Dipl.-Landw., Hardenbrugstrasse 19, D-6703 Limburgerhof (DE)**

**EP 0 056 087 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft die Verwendung von α-Azolylglykolen, zur Regulierung des Pflanzenwachstums.

Es ist bekannt, das 2-Chlorethyl-trimethylammoniumchlorid (Chlorcholinchlorid, CCC) (J. Biol. Chem., 235, 475 (1960)) zur Beeinflussung des Pflanzenwachstums zu verwenden. Mit seiner Hilfe läßt sich z. B. eine Hemmung des Längenwachstums bei einigen Getreidearten und eine Hemmung des vegetativen Wachstums bei einigen anderen Kulturpflanzen erreichen. Die Wirkung dieses Stoffes ist jedoch, insbesondere bei niedrigen Aufwandmengen, nicht immer ausreichend und genügt den Anforderungen der Praxis nicht.

Weiterhin ist es bekannt, 1-(4'-Bromphenyl)-1-allyloxy-2-(1'',2'',4''-triazolyl-(1''))-ethan zur Regulierung des Pflanzenwachstums von Raps, Weizen, Hafer, Roggen und Gerste zu verwenden (DE-OS 2 650 831). Dessen Wirkung ist jedoch, vor allem bei niedrigen Aufwandmengen, nicht immer befriedigend.

Es wurde nun gefunden, daß α-Azolylglykole der Formel I

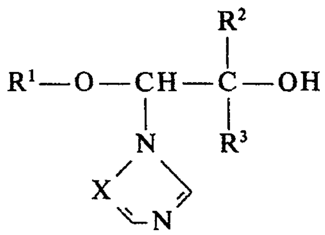

(I)

in welcher

- $R^1$ einen verzweigten oder unverzweigten Alkylrest mit 1 bis 4 C-Atomen,
- $R^2$ einen unverzweigten Alkylrest mit 1 bis 6 Kohlenstoffatomen oder einen verzweigten Alkylrest mit 3 bis 6 Kohlenstoffatomen oder einen unsubstituierten oder durch Halogen mono- oder disubstituierten Phenyl- oder Biphenylrest,
- $R^3$ Wasserstoff, einen unverzweigten Alkylrest mit 1 bis 6 Kohlenstoffatomen, einen verzweigten Alkylrest mit 3 bis 6 Kohlenstoffatomen, einen Alkenyl- oder Alkinylrest mit 2 bis 6 Kohlenstoffatomen oder gegebenenfalls durch Halogen mono- oder disubstituierten Benzylrest,
- X CH oder N bedeutet

sowie deren Salze und Metallkomplexe ausgezeichnet zur Beeinflussung des Pflanzenwachstums geeignet sind und eine sehr gute Pflanzenverträglichkeit zeigen.

Falls alle Verbindungen der Formel I sowie Verfahren zu ihrer Herstellung sind aus EP-A-0 021 345 bekannt.

Die α-Azolylglykole der Formel I besitzen im Acetal-C-atom und — falls $R^2$ und $R^3$ verschieden sind — im Carbinol-C-atom je ein Asymetriezentrum. Je nach der Beschaffenheit von $R^1$ kommen eventuell weitere Asymmetriezentren hinzu. Mit üblichen Trennmethoden können die Verbindungen in Form einheitlicher Enantiomerer bzw. Diastereomerer erhalten werden. In der Praxis kann man sowohl die einheitlichen Enantiomeren bzw. Diastereomeren wie auch die bei der Synthese üblicherweise anfallenden Gemische verwenden. Letztere werden bevorzugt verwendet.

Die α-Azolylglykole der Formel I lassen sich dadurch herstellen, daß man

a) ein Keton der Formel II

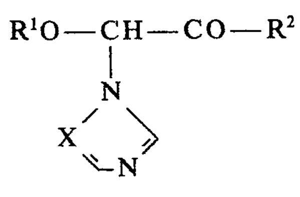

(II)

in der $R^1$, $R^2$ und X die oben erläuterten Bedeutungen haben, katalytisch oder mit komplexen Hydriden in Gegenwart eines Lösungsmittels und gegebenenfalls in Gegenwart eines Reaktionsbeschleunigers bei Temperaturen zwischen 0 und 100° C reduziert oder

b) ein Keton der Formel II im Grignardreagentien der Formel IV

$$R^3MgHal \qquad (IV)$$

in der $R^3$ die oben erläuterte Bedeutung hat und Hal für Chlor, Brom oder Jod steht, in Gegenwart

inerter Lösungsmittel bei Temperaturen zwischen 0 und 80° C umsetzt oder

c) ein Acetal der Formel V

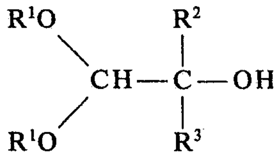

(V)

in der $R^1$, $R^2$ und $R^3$ die oben angegebenen Bedeutungen haben, mit anorganischen oder organischen Säurechloriden und anschließend mit einem Azol (Triazol, Imidazol) gegebenenfalls in Gegenwart eines Lösungsmittels und einer Base bei Temperaturen zwischen 0 und 100° C umsetzt.

Die Ausgangsverbindungen II sind herstellbar durch Umsetzung von $\alpha$-Halogenethern der Formel III

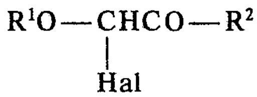

(III)

in der $R^1$ und $R^2$ die oben erwähnte Bedeutung haben und Hal, Chlor oder Brom bedeutet, mit Azolen (Triazol, Imidazol) oder deren Alkali- oder Erdalkalisalzen gegebenenfalls in Gegenwart eines Lösungsmittels und einer Base bei Temperaturen zwischen 0 und 100° C.

Die $\alpha$-Halogenether der Formel III können nach bekannten Methoden hergestellt werden (vgl. DE-OS 2 201 063 und B. Mylo, Chem. Ber. 44 (1911), S. 3212, Straus und Weber Ann. 498 (1932), S. 124). Ferner können die Verbindungen der Formel III auch durch Halogenierung (z. B. mit N-Bromsuccinimid) der $\alpha$-Alkoxyketone hergestellt werden.

Die Alkohole der Formel V sind teilweise bekannt wie zum Beispiel das 1,1-Dimethoxy-2-methylbutin-3-o1-2 (DE-PS 1 768 877), oder das 1,1-Dimethoxy-2-methylbuten-3-01-2 (DE-PS 1 115 238). Sie können aber auch nach bekannten Methoden hergestellt werden, indem man Ketone der Formel VI

$$(R^1O)_2CH-CO-R^2 \qquad (IV)$$

katalytisch oder mit komplexen Hydriden hydriert oder mit Grignardreagentien der Formel IV umsetzt.

Geeignete anorganische oder organische Säurehalogenide für das Verfahren c) sind beispielsweise Thionylchlorid, Acetylchlorid oder Acetylbromid. Darüber hinaus sind alle üblichen gut zugänglichen Säurehalogenide verwendbar.

Geeignete anorganische oder organische Basen, die gegebenenfalls auch als säurebindende Mittel in die Verfahren a oder c eingesetzt werden können, sind beispielsweise Alkali- und Erdalkalihydroxide — wie Natriumhydroxid, Kaliumhydroxid, Calciumhydroxid —, Alkalicarbonate — wie Kalium- oder Natriumcarbonat—, Alkalihydride — wie Natriumhydrid—, Alkali- oder Erdalkalialkoholate — wie Natriummethylat, Magnesiummethylat oder Natriumisopropylat —, oder tertiäre Amine — wie Trimethylamin, Triethylamin, N,N-Dimethylanilin, N,N-Dimethylcyclohexylamin, N-Methylpiperidin oder Pyridin.

Ferner können die Azole (Triazol, Imidazol) selbst als Basen verwendet werden. Mit geeigneten Basen, wie zum Beispiel Alkalhydrid — wie Natriumhydrid—, Lithiumalkyl — wie Butyllithium —, oder Alkali- oder Erdalkalialkoholaten — wie Natriummethylat — können die Azole auch in einer vorgeschalteten Umsetzung zunächst in ihre Salze überführt und dann als solche zur Reaktion gebracht werden.

Zu den bevorzugten Lösungs- bzw. Verdünnungsmitteln gehören Halogenkohlenwasserstoffe — wie Methylenchlorid, Chloroform, 1,2-Dichlorethan, Chlorbenzol —, aliphatische oder aromatische Kohlenwasserstoffe — wie Cyclohexan, Petrolether, Benzol, Toluol, oder Xylole —, Ester — wie Essigsäureethylester—, Amide — wie Dimethylformamid —, Nitrile — wie Acetonitril —, Sulfoxide — wie Dimethylsulfoxid —, Ketone — wie Aceton oder Methylethylketon —, Ether — wie Diethylether, Tetrahydrofuran oder Dioxan —, oder entsprechende Gemische.

Als Reaktionsbeschleuniger kommen vorzugsweise Metallhalogenide — wie Kaliumiodid —, Kronenether, quartäre Ammoniumverbindungen — wie Tetrabutylammoniumiodid — oder Säuren oder Kombinationen dieser Reaktionsbeschleuniger in Frage.

Die Umsetzungen werden im allgemeinen bei Temperaturen zwischen 9 und 150° C in einem Zeitraum von 1 bis 60 Stunden durchgeführt, drucklos oder unter Druck, kontinuierlich oder diskontinuierlich.

Zur Isolierung der Verbindungen der Formel I wird nach üblichen Methoden vorgegangen. Im allgemeinen bedürfen die anfallenden Produkte keiner weiteren Reinigung, sie können

aber auch nach bekannten Methoden wie Umkristallisation, Extraktion, Destillation oder Chromatographie weiter gereinigt werden.

Falls gewünscht, werden die α-Azolylglykole der Formel I anschließend nach bekannten Verfahren in ihre für Pflanzen verträglichen Salze oder in ihre Metallkomplexe überführt.

Zur Salzbildung eignen sich beispielsweise: Salzsäure, Bromwasserstoffsäure, Schwefelsäure, Salpetersäure, Phosphorsäure, Essigsäure oder Dodecylbenzolsulfonsäure. Die Wirksamkeit der Salze geht auf das Kation zurück, so daß die Wahl des Anions beliebig ist.

Metallkomplexe bilden sich durch Anlagerung der neuen Verbindungen an die Kationen von Metallsalzen. Besonders eignen sich hierfür Kupfer(II)-chlorid, Kupfer(II)-sulfat, Kupfer(II)-nitrat, Zink(II)-chlorid, Eisen(III)-chlorid, Mangan(II)-chlorid, Nickel(II)-bromid.

Die folgenden Beispiele schildern die Herstellung der neuen Substanzen:

## Beispiel 1

### a) Herstellung des Ausgangsmaterials

Zu 48 g 1,1-Dimethoxy-3,3-dimethylbutan-2-on (vgl. J. B. Wright J. Am. Chem. Soc. 77 (1955), S. 4883) werden unter Rühren 36,8 g Acetylbromid getropft. Dabei steigt die Temperatur auf 53°C an. Nach einstündigem Rühren wird diese Lösung zu einer Lösung von 41,4 g Triazol in 100 ml Dimethylformamid und 100 ml Tetrahydrofuran getropft. Es wird drei Stunden gerührt, dann das Reaktionsgemisch eingeengt, in Methylenchlorid aufgenommen und dreimal mit je 50 ml Wasser gewaschen. Die organische Phase wird abgetrennt, getrocknet und eingeengt. Das verbleibende Öl wird über eine Kolonne destilliert. Zwischen 84 und 86°C/0,1 mbar gehen 44 g 1-(1′,2′,4′-Triazol-1′-yl)-1-methoxy-3,3-dimethyl-butan-2-on über.

### b) Herstellung des Endproduktes

39,4 g 1-(1′,2′,4′-Triazol-1′-yl)-1-methoxy-3,3-dimethylbutan-2-on werden in 80 ml Methanol bei 10—20°C portionsweise mit 4,5 g Natriumborhydrid versetzt. Anschließend wird 1 Stunde bei Rückflußtemperatur gerührt. Das Reaktionsgemisch wird in 80 ml Wasser eingerührt und dreimal mit je 100 ml Methylenchlorid extrahiert. Die organische Phase wird abgetrennt, getrocknet und eingeengt. Das verbleibende Öl kristallisiert aus Petrolether aus. Man erhält so 34 g 1-(1′,2′,4′-Triazol-1′-yl)-1-methoxy-3,3-dimethylbutanol-2 vom Schmelzpunkt 62—64°C.

| | | | |
|---|---|---|---|
| ber.: | C 54,3 | H 8,6 | N 21,1 |
| gef.: | C 54,5 | H 8,4 | N 21,2. |

## Beispiel 2

Zu einer Lösung von 0,2 Mol 4-Chlorphenylmagnesiumbromid (hergestellt aus 4,9 g Magnesium und 38,3 g 4-Bromchlorbenzol) in 150 ml Ether wird eine Lösung von 1-(1′,2′,4′-Triazol-1′-yl)-1-methoxyaceton in 100 ml Ether getropft. Anschließend wird 5 Stunden bei Rückflußtemperatur gerührt. Zu dem abgekühlten Reaktionsgemisch werden 50 g Eis und dann 25%ige wäßrige Ammoniumchloridlösung zugetropft, bis sich die Phasen klar trennen. Die organische Phase wird abgetrennt und die wäßrige Phase zweimal mit je 100 ml Ether extrahiert. Die vereinigten Etherphasen werden mit Wasser neutral gewaschen, getrocknet und eingeengt. Aus Petrolether erhält man 23 g kristallines 1-(1′,2′,4′Triazol-1′-yl)-1-methoxy-2-(4′-chlorphenyl)-propanol-2 vom Schmelzpunkt 80—82°C.

## Beispiel 3

Zu 14,8 g 1,1-(Dimethoxy)-2-methyl-butanol-2 werden unter Rühren 12,3 g Acetylbromid getropft. Dabei steigt die Temperatur bis 60°C an. Nach einer Stunde Rühren wird dieses Reaktionsgemisch zu einer Lösung von 13,8 g Triazol in 100 ml Tetrahydrofuran und 50 ml Dimethylformamid getropft. Nach dem Rühren über Nacht wird das Reaktionsgemisch eingeengt, in Methylenchlorid aufgenommen und dreimal mit je 50 ml Wasser gewaschen. Die organische Phase wird getrocknet und eingeengt. Das verbleibende Öl wird destilliert. Bei 95—110°C/0,4 mbar gehen 6 g 1-(1′,2′,4′-Triazol-1′-yl)-1-methoxy-2-methylbutanol-2 über.

Analog Beispielen 1—3 können folgende α-Azolylglykole der Formel I hergestellt werden:

| Nr. | $R^1$ | $R^2$ | $R^3$ | X | Physikal. Konst. |
|---|---|---|---|---|---|
| 4 | $CH_3$ | $2{,}4\text{-}Cl_2-C_6H_3-$ | H | N | Fp 136–138° C |
| 5 | $C_2H_5$ | $2{,}4\text{-}Cl_2-C_6H_3-$ | H | N | Fp 113–115° C |
| 6 | $CH_3$ | $C_6H_5-$ | $CH_3$ | N | Fp 102–104° C |
| 7 | $n\text{-}C_4H_9$ | $4\text{-}Cl-C_6H_4-$ | $CH_3$ | N | Fp 58–70° C |
| 8 | $iso\text{-}C_4H_9$ | $4\text{-}Cl-C_6H_4-$ | $CH_3$ | N | Öl |
| 9 | $CH_3$ | $4\text{-}(C_6H_5)-C_6H_4-$ | $CH_3$ | N | Fp 176–178° C |
| 10 | $CH_3$ | $CH_3$ | $4\text{-}Cl-C_6H_4-CH_2-$ | N | Fp 110–112° C |
| 11 | $CH_3$ | $2{,}4\text{-}Cl_2-C_6H_3-$ | $CH_3$ | N | Fp 135–137° C |
| 12 | $C_2H_5$ | $2{,}4\text{-}Cl_2-C_6H_3-$ | $CH_3$ | N | Fp 150–152° C |
| 13 | $CH_3$ | $CH_3$ | H | N | Fp 140–142° C |
| 14 | $CH_3$ | $CH_3$ | H | CH | |
| 15 | $CH_3$ | $tert\text{-}C_4H_9-$ | $CH_3$ | N | Fp 85–86° C |
| 16 | $CH_3$ | $tert\text{-}C_4H_9-$ | $CH=CH_2$ | N | Fp 85° C |
| 17 | $CH_3$ | $tert\text{-}C_4H_9-$ | $CH_2-C_6H_5$ | N | Fp 90–92° C |
| 18 | $CH_3$ | $tert\text{-}C_4H_9-$ | $4\text{-}Cl-C_6H_4-CH_2-$ | N | Fp 94–96° C |
| 19 | $n\text{-}C_4H_9$ | $tert\text{-}C_4H_9-$ | $CH_3$ | N | $Kp_{0,01}$ 116–120° C |
| 20 | $n\text{-}C_4H_9$ | $tert\text{-}C_4H_9-$ | H | N | $Kp_{0,2}$ 100–125° C |
| 21 | $CH_3$ | $2{,}4\text{-}Cl_2-C_6H_3-$ | H | CH | Öl |
| 22 | $C_2H_5$ | $2{,}4\text{-}Cl_2-C_6H_3-$ | H | CH | Öl |
| 23 | $CH_3$ | $C_6H_5-$ | $CH_3$ | CH | |
| 24 | $n\text{-}C_4H_9$ | $4\text{-}Cl-C_6H_4-$ | $CH_3$ | CH | |
| 25 | $CH_3$ | $2{,}4\text{-}Cl_2-C_6H_3$ | $CH=CH_2$ | CH | |
| 26 | $CH_3$ | $4\text{-}(C_6H_5)-C_6H_4-$ | $CH_3$ | CH | |
| 27 | $CH_3$ | $4\text{-}Cl-C_6H_4$ | $CH_3$ | CH | Öl |
| 28 | $C_2H_5$ | $2{,}4\text{-}Cl_2-C_6H_3-$ | $4\text{-}Cl-C_6H_4-CH_2-$ | CH | |
| 29 | $CH_3$ | $tert\text{-}C_4H_9-$ | $CH_3$ | CH | |
| 30 | $CH_3$ | $tert\text{-}C_4H_9-$ | H | CH | |
| 31 | $CH_3$ | $tert\text{-}C_4H_9-$ | $CH=CH_2$ | CH | |
| 32 | $n\text{-}C_4H_9$ | $tert\text{-}C_4H_9-$ | $CH_2-C_6H_5$ | CH | |
| 33 | $n\text{-}C_4H_9$ | $tert\text{-}C_4H_9-$ | $CH_3$ | CH | |
| 34 | $n\text{-}C_4H_9$ | $tert\text{-}C_4H_9-$ | $CH=CH_2$ | CH | |
| 35 | $CH_3$ | $2{,}4\text{-}Cl_2-C_6H_3$ | $CH_3$ | CH | |

Fortsetzung

| Nr. | $R^1$ | $R^2$ | $R^3$ | X | Physikal. Konst. |
|---|---|---|---|---|---|
| 36 | $C_2H_5$ | $2,4\text{-}Cl_2-C_6H_3$ | $CH_3$ | CH | Öl |
| 37 | $n\text{-}C_3H_7$ | $2,4\text{-}Cl_2-C_6H_3$ | $CH_3$ | CH | Öl |
| 38 | $CH_3$ | $4\text{-}Cl-C_6H_4$ | H | CH | |
| 39 | $C_2H_5$ | $4\text{-}Cl-C_6H_4$ | H | CH | |

Die Verbindungen der Formel I können praktisch alle Entwicklungsstadien einer Pflanze verschiedenartig beeinflussen und werden deshalb als Mittel zur Beeinflussung des Pflanzenwachstums eingesetzt. Ihre Wirkungsvielfalt hängt ab vor allem

a) von der Pflanzenart und -sorte,
b) von dem Zeitpunkt der Applikation, bezogen auf das Entwicklungsstadium der Pflanze, und von der Jahreszeit,
c) von dem Applikationsort und -verfahren (Samenbeize, Bodenbehandlung oder Blattapplikation),
d) von den geoklimatischen Faktoren, z. B. Sonnenscheindauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der Pflanzenwachstumsregulatoren im Pflanzenbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in der Reduzierung des Längswachstums äußert. Die behandelten Pflanzen weisen demgemäß einen gedrungenen Wuchs auf; außerdem ist eine dunklere Blattfärbung zu beobachten.
Als vorteilhaft für die Praxis erweist sich z. B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.
Von wirtschaftlichem Interesse ist auch die Erhöhung der Standfestigkeit von lageranfälligen Kulturen wie Getreide, Reis, Mais und Soja. Die dabei verursachte Halmverkürzung und Halmverstärkung verringert oder beseitigt die Gefahr des »Lagerns« (des Umknickens) von Pflanzen unter ungünstigen Witterungsbedingungen vor der Ernte.
Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
Durch Anwendung der Mittel kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.
Mit den Mitteln läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z. B. Wintergetreide, ist es vorteilhaft, wenn die Bestände durch Behandlung mit Verbindungen der Formel I im Herbst zwar gut bestockt, aber nicht zu üppig in den Winter hineingehen. Dadurch kann der erhöhten Frostempfindlichkeit und — wegen der relativ geringen Blatt- bzw. Pflanzenmassen — dem Befall mit verschiedenen, insbesondere pilzlichen, Krankheiten vorgebeugt werden. Die Hemmung des vegetativen Wachstums ermöglicht außerdem bei vielen Kulturpflanzen eine dichtere Bepflanzung, so daß ein Mehrertrag, bezogen auf die Bodenfläche, erzielt werden kann.

B. Mit den Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zu vermehrtem Latexfluß zu stimulieren.
Dabei können die Stoffe Ertragssteigerungen durch Eingriffe in den pflanzlichen Stoffwechsel

bzw. durch Förderung oder Hemmung des vegetativen und/oder des generativen Wachstums verursachen.

C. Mit den Mitteln lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngeweben zwischen Frucht- bzw. Blatt und Sproßteil der Pflanze ist auch für ein gut kontrollierbares Entblättern der Bäume wesentlich.

Die Wirkung der Verbindungen der Formel I ist besser als bei bekannten Wachstumsregulatoren. Die Wirkung zeigt sich sowohl bei Monokotylen, z. B. Getreide wie Weizen, Gerste, Roggen, Hafer und Reis oder Mais als auch bei Dikotylen (z. B. Sonnenblumen, Tomaten, Soja, Reben, Baumwolle und vor allem Raps) und verschiedene Zierpflanzen wie Chrysanthemen, Poinsettien und Hibiskus.

Die $\alpha$-Azolylglykole können den Kulturpflanzen sowohl vom Samen her (als Saatgutbeizmittel) als auch über den Boden, d. h. durch die Wurzel, sowie durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g benötigt.

Für die Blatt- und Bodenbehandlung sind im allgemeinen Gaben von 0,01 bis 12 kg/12 kg/ha, bevorzugt 0,25 bis 3 kg/ha, als ausreichend zu betrachten.

Die Verbindungen der Formel I können im Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin), Dimethylformamid und Wasser; feste Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate) und Dispergiermittel wie Lignin, Sulfitablaugen und Methylcellulose.

Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Die Beispiele A—K erläutern die Herstellung von Formulierungen:

### Beispiel A

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

### Beispiel B

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an Salz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 10 000 Gewichtsteilen Wasser erhält man eine wäßrige Dispersion, die 0,02 Gew.-% des Wirkstoffs enthält.

Beispiel C

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel D

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel E

20 Gewichtsteile des Wirkstoffs 3 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel F

3 Gewichtsteile der Verbindung 3 werden mit 97 Gewichtsteilen feinteiligem Kaolin innig vermischt. Man erhält auf diese Weise ein Stäubemittel, das 3 Gew.-% des Wirkstoffs enthält.

Beispiel G

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel H

40 Gewichtsteile des Wirkstoffs 1 werden mit 10 Teilen Natriumsalz eines Phenolsufonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

Beispiel K

20 Teile des Wirkstoffs 2 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Eine erfindungsgemäße Verwendung kann in diesen Aufwendungsformen auch zusammen mit anderen Wirkstoffen vorliegen, wie z. B. mit Herbiziden, Insektiziden, Wachstumsregulatoren und Fungiziden oder auch mit Düngemitteln. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d. h. die Wirksamkeit des Kombinationsproduktes ist größer als die addierten Wirksamkeiten der Einzelkomponenten.

Die Wirkung der erfindungsgemäß verwendbaren Stoffe als Pflanzenwachstumsregulatoren wird beispielsweise wie folgt ermittelt:

Zur Bestimmung der wachstumsregulierenden Eigenschaft der Prüfsubstanzen wurden Testpflanzen (Sommergerste, Sorte »Union« und Sommerraps, Sorte »Petranova«) auf ausreichen mit Nährstoffen versorgtem Boden in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz dienten 2-Chlorethyl-trimethylammoniumchlorid und 1-(4′-Bromphenyl)-1-allyloxy-2-(1″, 2″, 4″-triazol-(1″))-ethan.

In diesen Versuchen zeigten insbesondere die Substanzen der Beispiele 2, 4, 5, 11, 17 und 19 eine deutlich bessere Wirkung als die Vergleichssubstanzen.

Gleichlaufend zur Reduzierung des Längenwachstums stieg die Farbintensität der Blätter an. Der erhöhte Chlorophyllgehalt läßt eine ebenfalls erhöhte Photosyntheserate und mit eine erhöhte Ertragsbildung erwarten.

## Patentansprüche

1. Verwendung eines α-Azolylglykoles der Formel I

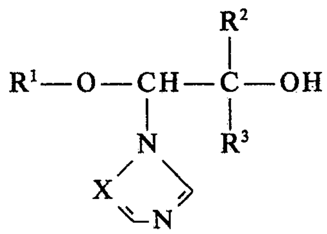

(I)

in welcher

$R^1$ einen verzweigten oder unverzweigten Alkylrest mit 1 bis 4 C-Atomen,

$R^2$ einen unverzweigten Alkylrest mit 1 bis 6 Kohlenstoffatomen oder einen verzweigten Alkylrest mit 3 bis 6 Kohlenstoffatomen oder einen unsubstituierten oder durch Halogen mono- oder disubstituierten Phenyl- oder Biphenylrest,

$R^3$ Wasserstoff, einen unverzweigten Alkylrest mit 1 bis 6 Kohlenstoffatomen, einen verzweigten Alkylrest mit 3 bis 6 Kohlenstoffatomen, einen Alkenyl- oder Alkinylrest mit 2 bis 6 Kohlenstoffatomen oder gegebenenfalls durch Halogen mono- oder disubstituierten Benzylrest

X CH oder N bedeutet

sowie dessen für Pflanzen verträglichen Salzen und Metallkomplexen als Mittel zur Beeinflussung des Pflanzenwachstums.

2. Verfahren zur Beeinflussung des Pflanzenwachstums, dadurch gekennzeichnet, daß man eine Verbindung wie in Anspruch 1 beschrieben auf Pflanzen oder deren Lebensraum oder auf Saatgut einwirken läßt.

## Claims

1. The use of an α-azolylglycol of the formula I

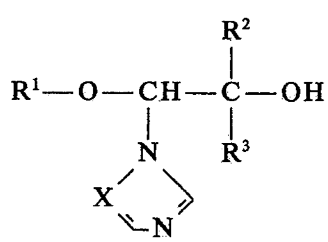

(I)

where $R^1$ is branched or linear alkyl of 1 to 4 carbon atoms, $R^2$ is linear alkyl of 1 to 6 carbon atoms or branched alkyl of 3 to 6 carbon atoms, unsubstituted phenyl or biphenyl, or phenyl or biphenyl mono- or disubstituted by halogen, $R^3$ is hydrogen, linear alkyl of 1 to 6 carbon atoms, branched alkyl of 3 to 6 carbon atoms, alkenyl or alkynyl of 2 to 6 carbon atoms, or benzyl optionally mono- or disubstituted by

halogen, and X is CH or N, and its plant- tolerated salts and metal complexes as agents for influencing plant growth.

2. A process for influencing plant growth, wherein a compound as claimed in claim 1 is allowed to act on plants, their habitat or the seed from which they are grown.

**Revendications**

1. Utilisation d'un $\alpha$-azolyl-glycol de la formule I

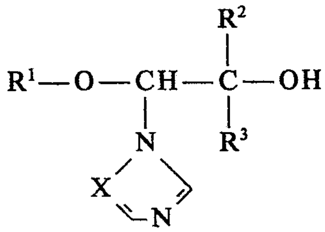

(I)

dans laquelle

$R^1$ désigne un radical alkyle ramifié ou non en $C_1$ à $C_4$,
$R^2$ désigne un radical alkyle non ramifié en $C_1$ à $C_6$ ou un radical alkyle ramifié en $C_3$ à $C_6$ ou un groupe phényle ou biphényle non substitué ou mono- ou di-substitué par de l'halogène,
$R^3$ désigne un atome d'hydrogène, un radical alkyle non ramifié en $C_1$à $C_6$, un radical alkyle ramifié en $C_3$ à $C_6$, un groupe alcényle ou alcynyle en $C_2$ à $C_6$ ou un groupe benzyle éventuellement mono- ou di-substitué par de l'halogène et
X est CH ou N,

ainsi que des sels et (ou) complexes de métaux d'un tel composé, compatibles avec le plantes, en tant que substances capables d'influencer la croissance de plantes.

2. Procédé pour influencer la croissance de plantes, caractérisé en ce que l'on fait agir un composé selon la revendication 1 sur les plantes, sur leur biotope ou sur les semences.